# EUROPÄISCHE PATENTANMELDUNG

(11) **EP 0 976 765 A1**
(43) Veröffentlichungstag der Anmeldung: **02.02.2000**
(21) Anmeldenummer: 99112237.5
(22) Anmeldetag: 25.06.1999
(51) Int. Cl.: C07K 14/745, C07K 14/765, A61L 2/00

(54) **Verfahren zur Herstellung einer Proteinlösung**

(30) Priorität: 10.07.1998 DE 19830914
(71) Anmelder: Centeon Pharma GmbH, 35002 Marburg (DE)
(72) Erfinder: Römisch, Jürgen, Dr., 35041 Marburg (DE); Weisse, Jörg, 35083 Ebsdorfergrund (DE); Veron, Jean-Luc, Dr., 69110 Sainte-Foy-Les-Lyon (FR); Djordjevich, Juliana, Chicago III. 60613 (US); Freudenberg, Wilfried, Dr., 35091 Cölbe (DE); Grandgeorge, Michael, Dr., 35041 Marburg (DE); Kalina, Barbara, Dr., 35043 Marburg (DE); Kappus, Peter, Dr., 35041 Marburg (DE); Konrad, Manfred, 1210 Wien (AT); Gregory, Rood S., Bourbonnais, III. 60914-9793 (US); Thill, Werner, Bourbonnais, III. 60914 (US)

(57) **Zusammenfassung**

Es wird ein Verfahren zur Herstellung einer Proteinlösung beschrieben, die selbst bei einer Hitzebehandlung nicht zur Flocken- oder Partikelbildung neigt, wobei die Hitzebehandlung in einem Glasgefäß erfolgt, das zuvor mit einer Detergenzlösung und anschließend mit destilliertem Wasser oder einem anderen detergenzfreien Lösungsmittel ausgespült wird.

## Beschreibung

Gegenstand der Erfindung ist ein Verfahren zur Herstellung einer Proteinlösung, die selbst bei einer Hitzebehandlung nicht zur Flocken- oder Partikelbildung neigt.

Es ist bekannt, daß in proteinhaltigen Lösungen häufig Partikel oder Flocken gebildet werden. Besonders bei Präparaten, die dem Patienten parenteral verabreicht werden, stellt die Flocken- oder Partikelbildung ein ernstes Problem dar. Diese Partikel müssen mit einem eigens dafür bereitgestellten Filter vor der Applikation entfernt werden oder, wenn dies nicht möglich ist, muß das Präparat verworfen werden. Die Gründe für eine solche Partikelbildung sind häufig unklar, jedoch wird dieses Phänomen besonders bei der Hitzebehandlung von Proteinlösungen beobachtet. Am Beispiel des Albumins kann dies verdeutlicht werden:

Albumin, das durch Reinigung aus humanem Plasma hergestellt wird, wird seit langem Patienten in Notfällen zur Substitution des Blutvolumens verabreicht. Das Albumin wird üblicherweise aus humanem Plasma durch fraktionierte Alkoholfällungen in der Kälte, durch die andere Plasmakomponenten abgetrennt werden, gewonnen. Üblicherweise werden zur arzneilichen Anwendung Albuminlösungen verwendet, die dieses Protein in Mengen von 5, 20 oder 25% enthalten. Um das Risiko einer Infektion der Patienten durch Bakterien oder Viren auszuschalten, die in einer kontaminierten Albuminlösung enthalten sein könnten, wird die Albuminlösung regelmäßig einer Hitzebehandlung im Endgefäß unterworfen. In Anwesenheit stabilisierender Zusätze wird die Albuminlösung dabei pasteurisiert, beispielsweise durch Erhitzen für mindestens 10 Stunden auf etwa 60°C.

Bei der Pasteurisierung kann es zur Bildung von flockenartigen Partikeln kommen, die bei leichtem Schütteln der Abfüllungen als mehr oder weniger große, opaleszierende und/oder weiße Konglomerate auffällig werden. Da die parenterale Applikation solcher Partikel vermieden werden muß, ist es erforderlich, entsprechende Abfüllungen auszusondern und zu verwerfen. Die Filtration einer partikelhaltigen Lösung vor der Verabreichung ist nicht praktikabel, weil die Filtration der Proteinlösung durch einen Sterilfilter durch ihre relativ hohe Viskosität erschwert wird.

Da die durch Proteinaggregation entstehenden Partikel sich vor allem während des Pasteurisierungsverfahrens bilden und nachträglich nicht aus der Endabfüllung entfernt werden können, hat man bereits versucht, durch Zusatz geeigneter Mittel die Flockenbildung zu reduzieren oder ganz zu verhindern. So ist in der europäischen Patentschrift 0 341 103 ein Verfahren zum Stabilisieren von Human-Albuminlösungen in einem Behälter mit Hilfe eines Stabilisierungsmittels beschrieben, bei dem man vor der Wärmebehandlung zusätzlich zu dem Stabilisierungsmittel ein oberflächenaktives Mittel wie Tween® 80, Tween® 20, Pluronic® F68 oder das Laurat von Polyethylenglykol 600 zusetzt. Hierbei beobachtet man ab einer bestimmten Detergenzkonzentration eine deutlich reduzierte Flockenanzahl, die bei Erhöhung des Detergenzgehaltes noch weiter vermindert oder ganz verhindert wird. Nachteilig ist jedoch, daß das Detergenz in der Proteinlösung verbleibt und somit zusammen mit dem Albumin dem Patienten appliziert wird. Obwohl sich bestimmte Detergenzien in bestimmten Konzentrationsgrenzen durchaus zur Applikation eignen, weil sie vertragen und im Organismus abgebaut werden können, wird ein Präparat ohne oder mit einem möglichst geringen Detergenzgehalt bevorzugt und angestrebt.

Es stellte sich deshalb die Aufgabe, ein Verfahren zu entwickeln, das die Herstellung einer mit einem Detergenz nicht oder nur ganz wenig belasteten Proteinlösung erlaubt, die selbst bei einer Hitzebehandlung nicht zur Flocken- oder Partikelbildung neigt.

Es wurde nun gefunden, daß eine derartige Proteinlösung hergestellt werden kann, wenn die Hitzebehandlung in einem Glasgefäß erfolgt, das zuvor mit einer Detergenzlösung und anschließend mit destilliertem Wasser oder einem anderen detergenzfreien Lösungsmittel ausgespült wird.

Es ist überraschend, daß das Einfüllen einer detergenzhaltigen Lösung in das zur Aufnahme der Proteinlösung vorgesehene Glasgefäß die Flocken- oder Partikelmenge der anschließend in dieses Gefäß gefüllten Albuminlösung bei der nachfolgenden Hitzebehandlung reduziert oder verhindert, obwohl das Gefäß vor dem Einfüllen der Proteinlösung mit destilliertem Wasser oder einem anderen detergenzfreien Lösungsmittel ausgespült wird, so daß nur noch Spuren oder überhaupt kein Detergenz mehr in der pasteurisierten Proteinlösung nachweisbar sind, trotzdem aber die Partikelrate signifikant erniedrigt wird.

Als Detergenzien eignen sich dem Fachmann bekannte Substanzen. Besonders bevorzugt sind dabei nicht-ionische Detergenzien, die unter den Handeisbezeichnungen Tween®, Triton® und Pluronic® bekannt sind. Dabei ist der Konzentration des Detergenz in der Lösung, die mit dem Glasgefäß in Kontakt gebracht wird, nach oben lediglich eine Grenze durch die Löslichkeit in der Flüssigkeit gesetzt. Konzentrationen unter 0,000001 Gew.-% sollten in Lösungen jedoch nicht unterschritten werden. Die innere Oberfläche des Glasgefäßes sollte möglichst vollständig benetzt werden, wobei die Inkubationsdauer nicht limitiert ist. Mit dem anschließenden Spülen des Gefäßes mit Wasser oder einer detergenzfreien Lösung soll erreicht werden, daß das Detergenz in der gebrauchsfertigen Proteinlösung nur in Spuren, vorzugsweise in einer Konzentration von weniger als 0,0002 Gew-% vorhanden oder gar nicht mehr nachweisbar ist. Diese Behandlung des Glasgefäßes führt zu dem unerwarteten Ergebnis, daß die Partikelrate nach der Pasteurisierung in der abgefüllten Proteinlösung ganz gering ist, wie es das nachfolgende Beispiel zeigt.

Die Ursache für die Bildung von Flocken oder Partikeln während der Hitzebehandlung in einer Proteinlösung ist noch nicht vollständig verstanden worden, jedoch lassen die Ergebnisse der vorliegenden Erfindung vermuten, daß die innere Oberfläche des Gefäßes hierbei ein wichtiger Faktor ist. Das Detergenz könnte durch Absättigung entsprechender Interaktionsstellen, die als "Partikelkeimzentren" wirken, die Flockenentstehung verhindern. Besonders vorteilhaft ist bei dem erfindungsgemäßen Verfahren, daß kein oder nur Spuren von Detergenz in Lösung gehen und somit das Präparat wenig oder überhaupt nicht belasten.

Durch das erfindungsgemäße Verfahren kann die Flocken- oder Partikelbildung in den unterschiedlichsten Proteinlösungen verhindert werden. Ganz besonders bewährt hat sich das Verfahren bei der Stabilisierung von Albuminlösungen sowie bei Lösungen von Blutgerinnungsfaktoren. Die Proteine können entweder aus natürlichem Material isoliert oder rekombinant oder transgen hergestellt sein. Die Erfindung läßt sich mit allen gebräuchlichen Behälterglasarten realisieren, insbesondere mit solchen aus Typ I- und Typ II-Glas.

Nicht in jedem Fall tritt eine Flocken- oder Partikelbildung in Proteinlösung erst während einer Hitzebehandlung ein. Manchmal wird auch schon bei Zimmertemperatur oder bei längerer Lagerung eine Partikelbildung beobachtet, die durch das erfindungsgemäße Verfahren verhindert oder vermindert werden kann. Ganz allgemein läßt sich sagen, daß die durch Gefäßoberflächen induzierten Bildungen von Proteinoligomeren, -dimeren oder -multimeren erfindungsgemäß reduziert werden können.

Das beschriebene Verfahren wird am Beispiel der Partikelbildung von Albumin während der Pasteurisierung näher erläutert:

### Beispiel:

Glasgefäße (50 ml Volumen) wurden mit einer Lösung, die Tween® 80 in einer Konzentration von 0,1% enthielt, randvoll gefüllt und für 30 Minuten bei Raumtemperatur inkubiert. Die Lösung wurde durch Ausgießen entfernt und die Gefäße auf dem Kopf stehend abtropfen gelassen. Danach wurden sie ein- bis dreimal im Wasser, geeignet für Injektionszwecke (WFI), gespült. Dazu wurden die Gefäße wiederum vollständig gefüllt und danach entsprechend direkt wieder geleert. Nach Lufttrocknen der Gefäße wurde eine 20%-ige Albuminlösung eingefüllt, die Gefäße mit Stopfen verschlossen und anschließend für 10 Stunden bei 60°C im Wasserbad inkubiert. Dabei wurden die Gefäße so im Wasserbad plaziert, daß eine hohe Partikelrate induziert wurde. Als Kontrollansätze dienten Gefäße, die nicht mit Detergenzlösungen vorbehandelt wordem waren. Als Positiv-kontrollen wurde ein Teil der Albuminlösung mit Tween 80 versetzt und in unbehandelte Gefäße gefüllt. Dabei wurde Detergenz zugesetzt, so daß die Endkonzentrationen in den entsprechenden Albuminlösungen zwischen 0,0001% und 0,1% lagen.

Die Anzahl partikelhaltiger Gefäße wurde jeweils durch optische Kontrolle bestimmt. Die Ergebnisse (Tabelle) zeigen, daß in 60% der Kontrollgefäße (Gefäße nicht mit Detergenz vorbehandelt) Partikel registriert wurden. Die Kontroll- abfüllungen (Albuminlösung mit 0,1% Tween) blieben erwartungsgemäß partikelfrei. Mit sinkender Konzentration an Tween 80 nahm die Partikelrate zu, wobei ein deutlicher diesbezüglicher Sprung zwischen 0,0025% und 0,001% zu verzeichnen war (siehe Tabelle). Die Gefäße, die mit detergenzhaltiger Lösung vorbehandelt und danach gespült worden waren, zeigten eine signifikant reduzierte Flockenrate gegenüber der Kontrolle, wobei nach einfachem Spülen lediglich 5% der Gefäße Partikel enthielten, nach zwei- und dreifachem Spülen zwischen 20 und 30%.

Die Gefäße wurden danach geöffnet, jeweils eine Probe wurde entnommen und die Tween-Konzentrationen quantifiziert. Dazu wurde ein modifiziertes Testsystem nach Thoma et al. (Sci. Pharm. 1964,3: 216-224) verwendet.

Während in den Kontrollen (ohne Tweenbehandlung) wie erwartet kein Detergenz nachweisbar war, konnten in den Lösungen, denen von 0,0001% bis 0,1% Tween zugesetzt worden war, die theoretisch erwarteten Konzentrationen in etwa detektiert werden (mit Ausnahme der außerhalb der Testgrenzen liegenden Gehalte).

Dagegen waren in den Lösungen, die den mit Detergenz vorbehandelten und danach gespülten Gefäßen entnommen worden waren, nur Spuren oder überhaupt kein Detergenz nachweisbar. Bereits nach zweimaligem Spülen der Gefäße lag der Tween 80-Gehalt der Albuminlösungen unter der Nachweisgrenze des Testsystems.

Vergleicht man nun die verschiedenen Ansätze, so fällt auf daß bei vergleichbar verminderter Partikelrate (also zum Beispiel etwa 30%), kein oder nur Spuren Tween in den vorbehandelten Gefäßen nachweisbar war (< 0,0002%), jedoch deutliche Detergenzmengen (0,002%) in den Ansätzen mit zugesetztem Tween (letzteres erwartungsgemäß). D.h., durch das beanspruchte Verfahren wird bei gleicher Reduktion der Partikelrate das Albumin mit einem mindestens 10-fach vermindertem Detergenzgehalt belastet.

**Tabelle**

| **Ansätze** | **Partikelrate (%) (n=10-25)** | **Tween 80 Konz. (%) Mittelwerte aus n=3-8** |
|---|---|---|
| **Kontrolle** | | |
| Proben/Gefäße ohne Tween 80 | 60 | < 0,0002 |

| **Proben + Tween 80 zugesetzt** | | |
|---|---|---|
| Konzentration Tween 80: | | |
| 0,0001 % | 60 | < 0,0002 |
| 0,001 % | 60 | 0,001 |
| 0,0025 % | 30 | 0,002 |
| 0,01 % | 20 | > 0,0012 |
| 0,1 % | 0 | > 0,0012 |

| Gefaß + Tween (0,1 %)-Vorbehandlung und anschließende Probenabfüllung | | |
|---|---|---|
| nach 1 x Spülen mit WFI | 5 | 0,0004 |
| nach 2 x Spülen mit WFI | 25 | < 0,0002 |
| nach 3 x Spülen mit WFI | 28 | < 0,0002 |

## Patentansprüche

1. Verfahren zur Herstellung einer Proteinlösung, die selbst bei einer Hitzebehandlung nicht zur Flocken- oder Partikelbildung neigt, dadurch gekennzeichnet, daß die Hitzbehandlung in einem Glasgefäß erfolgt, das zuvor mit einer Detergenzlösung und anschließend mit destilliertem Wasser oder einem anderen detergenzfreien Lösungsmittel ausgespült wird.

2. Verfahren nach Anspruch 1, dadurch gekennzeichnet, daß als Proteinlösung eine Albuminlösung oder eine Lösung von Blutgerinnungsfaktoren eingesetzt wird.

3. Verfahren nach den Ansprüchen 1 und 2, dadurch gekennzeichnet, daß die Hitzebehandlung als eine Pasteurisierung durchgeführt wird.

4. Verfahren nach den Ansprüchen 1 bis 3, dadurch gekennzeichnet, daß das Glasgefäß mit einer Lösung eines nicht-ionischen Detergenz ausgespült wird.

5. Verfahren nach den Ansprüchen 1 bis 4, dadurch gekennzeichnet, daß das Glasgefäß nach der Behandlung mit der Detergenzlösung wenigstens einmal mit destilliertem Wasser oder einem anderen detergenzfreien Lösungsmittel ausgespült wird.

6. Verfahren nach den Ansprüchen 1 bis 5, dadurch gekennzeichnet, daß die Detergenzkonzentration in der gebrauchsfertigen Proteinlösung < 0,0002% beträgt.

7. Proteinlösung, hergestellt nach den Ansprüchen 1 bis 6.
